(19) Europäisches Patentamt
European Patent Office
Office européen des brevets

(11) Publication number: **0 075 629 A1**

(12) **EUROPEAN PATENT APPLICATION**

(21) Application number: 81304514.3

(22) Date of filing: 29.09.81

(51) Int. Cl.³: C 07 D 233/90
A 61 K 31/415

(43) Date of publication of application: 06.04.83 Bulletin 83/14

(84) Designated Contracting States: AT BE CH DE FR GB IT LI NL SE

(71) Applicant: Sumitomo Chemical Industries Ltd.
15, Kitahama 5 chome
Higashi-ku, Osaka-shi(JP)

(72) Inventor: Atsumi, Toshio
3-45, Seiwadai Nishi, 2-chome
Kawanishi(JP)

(72) Inventor: Sanjiki, Tetsutaro
7-6, Funakicho
Ibaraki(JP)

(72) Inventor: Kiyohara, Takao
14-7, Mefu 2-chome
Takarazuka(JP)

(74) Representative: Geering, Keith Edwin et al,
REDDIE & GROSE 16 Theobalds Road
London WC1X 8PL(GB)

(54) **Imidazole derivatives, their preparation, and their pharmaceutical compositions.**

(57) Antitumor agents and immunosuppressants (I):

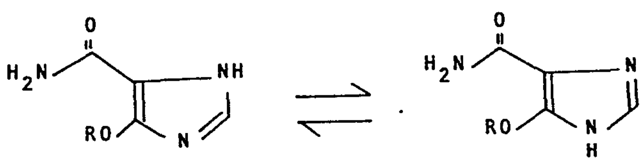
(1)

wherein R is a monosubstituted benzoyl group in which the substituent is (a) a phenyl, alkanoyl, formyl, halogenoalkyl, aralkyloxy, phenoxy, alkoxycarbonyl, aralkyloxycarbonyl alkanoyloxy, benzoyl, carboxyl, hydroxy, aralkyloxycarbonylamino, lower alkoxycarbonylamino, carboxyamino or carbamoyl group or a group (IV)

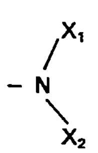
(IV)

(wherein $X_1$ and $X_2$ are each independently hydrogen or lower alkyl); (b) a cinnamoyl group which may be substituted at the α-position and/or the phenyl ring with one or more substituents selected independently from alkyl, alkoxy, aryl, nitro, methylenedioxy, formyl, halogenoalkyl, hydroxy, carboxyl, amino and cyano groups and halogens or (c) a benzoyl group substituted with from two to five substituents selected independently from alkyl, alkoxy, aralkyloxy, nitro, hydroxy, alkanoyloxy, formyl, carboxyl, alkylthio, alkylsulfonyl, sulfo and sulfamoyl groups, halogens, and groups (IV) above; and non-toxic salts thereof. They are obtained from 4-carbamoylimidazolium-5-olate and the corresponding acids ROH.

EP 0 075 629 A1

IMIDAZOLE DERIVATIVES, THEIR PREPARATION, AND THEIR PHARMACEUTICAL COMPOSITIONS

The present invention relates to 4-carbamoylimidazolium-5-olate derivatives, their preparation, and pharmaceutical compositions containing them.

The derivatives of the present invention are of formula (I) :

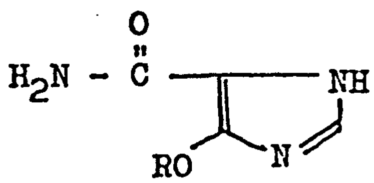

(I)

wherein R is a monosubstituted benzoyl group in which the substitutent is (a) a phenyl, alkanoyl, formyl, halogenoalkyl, aralkyloxy, phenoxy, alkoxycarbonyl, aralkyloxycarbonyl, alkanoyloxy, benzoyl, carboxyl, hydroxy, aralkyloxycarbonylamino, lower alkoxycarbonylamino, carboxyamino or carbamoyl group or a group (IV)

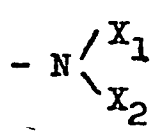

(IV)

(wherein $X_1$ and $X_2$ are each independently hydrogen or lower alkyl);(b) a cinnamoyl group which may be substituted at the α-position and/or the phenyl ring with one or more substitutents selected independently from alkyl, alkoxy, aryl, nitro, methylenedioxy, formyl, halogenoalkyl, hydroxy, carboxyl, amino and cyano groups and halogens or (c) a benzoyl group substituted with from two to five substituents selected independently from alkyl, alkoxy, aralkyloxy, nitro, hydroxy,

alkanoyloxy, formyl, carboxyl, alkylthio, alkylsulfonyl, sulfo and sulfamoyl groups, halogens, and groups (IV) above; and non-toxic salts thereof.

As used herein, the term "alkanoyl" comprehends lower alkanoyl having 2 to 6 carbon atoms (e.g. acetyl, propionyl, pivaloyl, hexanoyl), medium alkanoyl having 7 to 12 carbon atoms (e.g. lauroyl) and higher alkanoyl having 13 to 22 carbon atoms (e.g. octanoyl, palmitoyl).

The term "halogenoalkyl" means lower alkyl having 1 to 6 carbon atoms substituted with halogen(s), e.g. trifluoromethyl, β-chloroethyl.

The term "aralkyloxy" means lower alkoxy having 1 to 6 carbon atoms substituted with an aryl group, e.g. benzyloxy, α-methylbenzyloxy, phenethyloxy and the like.

The term "alkoxycarbonyl" comprehends lower alkoxy carbonyl having 2 to 7 carbon atoms (e.g. methoxycarbonyl, ethoxycarbonyl, n-propoxycarbonyl, isopropoxycarbonyl), medium alkoxycarbonyl having 8 to 13 carbon atoms (e.g. octyloxycarbonyl) and higher alkoxycarbonyl having 14 to 23 carbon atoms (e.g. octadecyloxycarbonyl, docosyloxycarbonyl).

The term "aralkyloxycarbonyl" means a carbonyl substituted with an above aralkyloxy group (e.g. benzyloxycarbonyl, α-methylbenzyloxycarbonyl, phenethyloxycarbonyl).

The term "alkanoyloxy" comprehends lower alkanoyloxy having 2 to 6 carbon atoms (e.g. acetoxy, propionyloxy, pivaloyloxy, hexanoyloxy), medium alkanoyloxy having 7 to 12 carbon atoms (e.g. octanoyloxy, lauroyloxy) and higher

alkanoyloxy having 13 to 22 carbon atoms (e.g. palmitoyloxy, stearoyloxy).

The term "aralkyloxycarbonylamino" means an amino group substituted with an above aralkyloxycarbonyl group.

The term "lower alkoxycarbonylamino" means an amino group substituted with an above lower alkoxycarbonyl group.

The term "alkyl" comprehends lower alkyl having 1 to 6 carbon atoms (e.g. methyl, ethyl, n-propyl, iso-propyl, n-butyl, isobutyl, t-butyl, hexyl), medium alkyl having 7 to 12 carbon atoms (e.g. octyl, decyl, dodecyl) and higher alkyl having 13 to 22 carbon atoms (e.g. pentadecyl, docosyl).

The term "alkoxy" comprehends lower alkoxy having 1 to 6 carbon atoms (e.g. methoxy, ethoxy, n-propoxy, isopropoxy, n-butoxy, isobutoxy), medium alkoxy having 7 to 12 carbon atoms (e.g. octyloxy, dodecyloxy) and higher alkoxy having 13 to 22 carbon atoms (e.g. octadecyloxy, docosyloxy).

The term " aryl" covers phenyl, tolyl, and naphthyl, etc.

The term "halogen" covers fluorine, chlorine, bromine and iodine.

The term "alkylthio" means an alkylthio substituted with an above alkyl group.

The term "alkylsulfonyl" means an alkylsulfonyl substituted with an above alkyl group.

The term "lower alkyl" in $X_1$ or $X_2$ means a lower alkyl having 1 to 6 carbon atoms as stated above.

The cinnamoyl group may be unsubstituted, or substituted (at the α-position and/or the phenyl ring) with one or more of the same or different substituents mentioned above; substituted groups included e.g. p-methylcinnamoyl, p-methoxycinnamoyl, α-phenylcinnamoyl, m-nitrocinnamoyl, 3,4-methylenedioxycinnamoyl, p-formylcinnamoyl, m-trifluoromethylcinnamoyl, p-chlorocinnamoyl, p-aminocinnamoyl, p-hydroxycinnamoyl, o-carboxycinnamoyl, α-methylcinnamoyl, α-fluorocinnamoyl, α-cyano-p-hydroxycinnamoyl, 2,6-dichlorocinnamoyl, 2,5-dimethoxycinnamoyl, 3,4-dimethylcinnamoyl, 4-hydroxy-3-methoxycinnamoyl, 3,4-dihydroxycinnamoyl, 2,4,5-trimethyoxycinnamoyl, and 3,5-dimethoxy-4-hydroxycinnamoyl.

Compounds of formula (I) can be prepared by reacting 4-carbamoylimidazolium-5-olate (II)

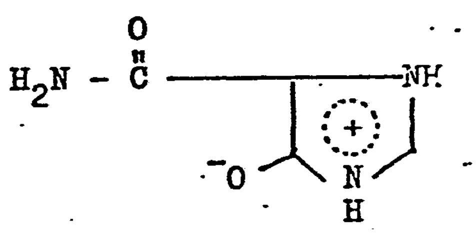

(II)

or reactive derivative thereof with carboxylic acid of formula (III);

$$R - OH \qquad (III)$$

(wherein R is as defined above) or reactive derivative thereof.

Examples of preferred reactive derivatives of carboxylic acids of the formula (III) are carboxylic acid halides (e.g. chlorides, bromides, iodides, fluorides),

anhydrides and mixed anhydrides (e.g. with ethyl chloroformate, isobutyl chloroformate and the like), activated esters (e.g. p-nitrophenyl ester, ester with N-hydroxysuccinimide), imidazolides (e.g. prepared by reacting N,N'-carbonyldiimidazole with carboxylic acid (III)), and activated intermediates prepared by reacting carboxylic acid (III) with reaction products obtained from N,N-dimethylformamide and oxalyl chloride (or phosgene or thionyl chloride or phosphorus pentachloride).

Examples of preferred reactive derivatives of 4-carbamoylimidazolium-5-olate of formula (II) are trimethylsilyl derivatives, trialkyltin derivatives, mercury salts, and silver salts.

Typical examples of preferred solvents which may be used in this reaction are methylene chloride, chloroform, pyridine, diethyl ether, tetrahydrofuran, dioxane, benzene, toluene, methanol, ethanol, N,N-dimethylformamide, formamide, N,N-dimethylacetamide, acetonitrile, nitromethane, acetone, ethyl acetate, dimethylsulfoxide, dichloromethane, dichloroethane, xylene and water.

The reaction can generally be effected at a reaction temperature from $-78^{o}$ to $100^{o}C.$, preferably from $-60^{o}$ to $60^{o}C.$

The reaction of 4-carbamoylimidazolium-5-olate with carboxylic acid halide can usually be carried out in an inert polar solvent or a mixture of water and inert organic solvent, preferably in the presence of an inorganic or organic base, at a temperature from -10° to 60°C. using one to two mole equivalents of the acid halide.

Typical examples of said inert polar solvent are tetrahydrofuran, dioxane, pyridine, N,N-dimethylformamide, formamide, N,N-dimethylacetamide and dimethylsulfoxide. Typical examples of said inert organic solvents are tetrahydrofuran, dioxane, diethyl ether, chloroform, dichloromethane, dichloroethane, benzene, toluene, and xylene. Examples of preferred inorganic base are sodium hydroxide, sodium carbonate, sodium bicarbonate, potassium carbonate or bicarbonate and potassium hydroxide. Examples of preferred organic base are pyridine, triethylamine and N,N-dimethylaniline.

The reaction of 4-carbamoylimidazolium-5-olate with activated intermediate prepared by reacting carboxylic acid (III) with reaction product obtained from N,N-dimethylformamide and oxalyl chloride (or phosgene or thionyl chloride or phosphorus pentachloride) can usually be carried out in an organic solvent (e.g. acetonitrile, pyridine, N,N-dimethylformamide, N,N-dimethylacetamide, chloroform) at a temperature from -78° to 80°C.

The compounds of formula (I) can also be prepared by reacting silylated derivative of 4-carbamoylimidazolium-5-olate with reactive derivative of carboxylic acid (III) (e.g. acid halide) at a temperature from -78° to 50°C. in an inert organic solvent (e.g. dimethylformamide, tetrahydrofuran, dioxane, diethyl ether, benzene, toluene).

The silylated derivatives of 4-carbamoylimidazolium-5-olate are known and can be prepared by known methods (Hayashi, et al. Japanese Patent Publication (Kokai) No. 50-121276). When a compound of formula (I) exists in the form of silylated derivative in the reaction mixture, the compound (I) can be obtained by a desilylation reaction with desilylating reagent(s) (e.g. acetic acid, methanol).

When an acid halide of acid (III) is used as a reactant the eliminated halide can be neutralized by organic base (e.g. triethylamine, pyridine).

Compounds of formula (I) substituted by any of amino, hydroxy and carboxyl groups can be prepared by the said acylation methods after protecting any substituent amino, hydroxy or carboxyl group with a protective group (e.g. benzyl, benzyloxycarbonyl, t-butoxycarbonyl and the like) and then removing the protective group(s).

The compounds of formula (I) can be isolated and purified by known purification methods (e.g. recrystallization, column chromatography).

The compounds of formula (I) may form salts with inorganic acids(e.g. hydrochloric acid, sulfuric acid, nitric acid, phosphoric acid) or organic acids(e.g. p-toluenesulfonic acid, benzenesulfonic acid, methanesulfonic acid, tartaric acid, malic acid, lactic acid, maleic acid, fumaric acid).

The imidazole derivatives of the present invention may exist in a mixture of two tautomers

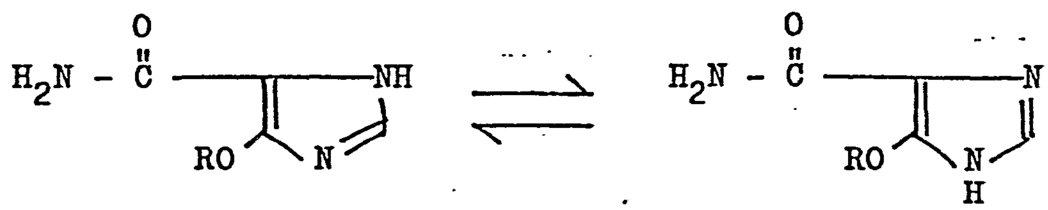

both of which are within the scope of the present invention.

Compounds of the present invention have shown potent antitumor activities against Sarcoma 180, Lewis lung carcinoma, Ehrlich carcinoma, P-388 leukemia and the like. Compounds of formula (I) used as antitumor agents have exhibited good inhibitory effects against tumors and also a prolonging effect on life span.

The antitumor activities of compounds of the present invention were estimated according to the methods described in "Cancer chemotheraphy reports" Part 3, Vol. 3, (No. 2) p.13 (1972). The results are given in the following Table 1.

Table 1 Antitumor effect on mouse experimental tumors

| Compound | Dose (mg/kg) Route i.p. | Schedule | Inhibition Ratio (%) Lewis lung carcinoma (solid) |
|---|---|---|---|
| 5-Carbamoyl-1H-imidazole-4-yl p-phenylbenzoate | 100 | 5q2d | 77.0 |
| 5-Carbamoyl-1H-imidazole-4-yl p-chlorocinnamate | 100 | 5q2d | 90 |
| 5-Carbamoyl-1H-imidazole-4-yl m-nitrocinnamate | 100 | 5q2d | 90 |

Table 1 (cont'd)

| | | | |
|---|---|---|---|
| 5-Carbamoyl-1H-imidazole-4-yl 3',4'-dimethoxy-benzoate | 100 | 5q2d | 83.1 |
| 5-Carbamoyl-1H-imidazole-4-yl 3'-methoxy-4'-methylbenzoate | 100 | 5q2d | 89.4 |

$BDF_1$ male mice, 5 weeks old, weighing between 18 and 22 grams were used. Each test group was composed of 6 to 7 mice. Two million cells of Lewis lung carcinoma were injected in the hind leg. The drug was administered intraperitoneally at days 1, 3, 5, 7 and 9 (or 5q2d).

After killing the mice at day 13, tumors were removed and weighed. The tumor inhibitory ratio was calculated according to the following formula:

$$\text{Inhibition ratio} = \left(1 - \frac{\text{the mean tumor weight of treated group}}{\text{the mean tumor weight of control group}}\right) \times 100$$

Compounds of the present invention have also shown immunosuppressive activity.

Compounds (I) of the present invention have shown low toxicity. They have shown no toxic symptons even on oral administration of over 1000 mg/kg to a mouse. Moreover, they have not exhibited the effect of decreasing peripheral leucocytes, which is one of the most serious side effects of prior immunosuppressants.

Compounds of the present invention can be administered orally or parenterally to warm-blooded animals at a daily dose of 2 - 200 mg/kg (as an antitumor agent) or 1 - 100 mg/kg (as an immunosuppresant agent) in conventional

unit dosage form.

Compounds of the present invention may be made up alone or together with conventional pharmaceutical carrier or diluent into a conventional solid or liquid pharmaceutical preparation (e.g. powders, granules, tablets, capsules, suspensions, emulsions, solutions) using methods conventional in the pharmaceutical field. For example, tablets or capsules may contain 50 - 500 mg of compound (I).

Especially, compounds (I) of the present invention can be used for oral administration and may be effective for a long period.

The following Examples are given to illustrate the present invention further but it is not intended to limit the present invention thereto.

Example 1

To a suspension of 0.636 g. of 4-carbamoylimidazolium-5-olate in 15 ml of dry pyridine was added dropwise 1.2 g of 2,6-dimethylbenzoyl chloride at a temperature below 5°C. in an $N_2$ atomosphere. After being stirred for two hours at 41 - 43°C., the reaction mixture was cooled to room temperature and 0.8 g of triethylamine was added, and separated crystals were filtered off. Then the filtrate was concentrated under reduced pressure, and separated crystals were filtered off, washed with toluene and ether and dried to give 0.689 g of 5-carbamoyl-1H-imidazole-4-yl 2',6'-dimethylbenzoate, m.p. 176 - 177°C.

Crude material was recrystallized from N,N-dimethylformamide and water. m.p.: 180 - 180.5°C.

$\nu_{max}^{nujol}$ ($cm^{-1}$): 3425, 3160, 1725, 1670, 1610

Elemental analysis:

| | C(%) | H (%) | N(%) |
|---|---|---|---|
| Calculated for $C_{13}H_{13}N_3O_3 \cdot 0.3H_2O$ | 59.00 | 5.18 | 15.88 |
| Found | 59.1 | 5.0 | 15.6 |

## Example 2

Following a procedure similar to that of Example 1 but using 0.636 g of 4-carbamoylimidazolium-5-olate and 1.30 g of 3,4-dimethoxybenzoyl chloride there was obtained 1.353 g of 5-carbamoyl-1H-imidazole-4-yl 3',4'-dimethoxybenzoate. m.p.: 209.5°C. (dec.)

Crude material was recrystallized from N,N-dimethylformamide and water. m.p.: 216.5°C.

$\nu_{max}^{nujol}$ ($cm^{-1}$): 3440, 3330, 3120, 1750, 1650, 1590

Elemental analysis:

| | C(%) | H(%) | N(%) |
|---|---|---|---|
| Calculated for $C_{13}H_{13}N_3O_5$ | 53.61 | 4.50 | 14.43 |
| Found | 53.38 | 4.43 | 14.30 |

## Example 3

Following a procedure similar to that of Example 1 but using 0.636 g of 4-carbamoylimidazolium-5-olate and 2.0 g of 2,4,6-trimcthoxybenzoyl chloride there was obtained 0.699 g of 5-carbamoyl-1H-imidazole-4-yl 2',4',6'-trimethoxybenzoate. m.p.: 174°C. (charred)

Crude material was recrystallized from N,N-dimethylformamide and water. m.p.: 184.5°C. (charred)

$\nu_{max}^{nujol}$ ($cm^{-1}$): 3410, 3330, 1750, 1670, 1610, 1590

Elemental analysis:

| | C(%) | H(%) | N(%) |
|---|---|---|---|
| Calculated for $C_{14}H_{15}N_3O_6$ | 52.34 | 4.71 | 13.08 |
| Found | 52.2 | 4.8 | 13.2 |

Example 4

Following a procedure similar to that of Example 1 but using 0.508 g of 4-carbamoylimidazolium-5-olate and 1.83 g of 3,4-bisbenzyloxybenzoyl chloride there was obtained 1.548 g of 5-carbamoyl-1H-imidazole-4-yl 3',4'-bisbenzyloxybenzoate. m.p.: 189.5 - 191.5°C.

Crude material was recrystallized from N,N-dimethylformamide and water. m.p.: 191.5 - 192.5°C.

$\nu_{max}^{nujol}$ ($cm^{-1}$): 3460, 3150, 1740, 1670, 1605

Elemental analysis:

| | C(%) | H(%) | N(%) |
|---|---|---|---|
| Calculated for $C_{25}H_{21}O_5N_3$ | 67.71 | 4.77 | 9.48 |
| Found | 67.4 | 4.9 | 9.3 |

Example 5

A suspension of 0.55 g of 5-carbamoyl-1H-imidazole-4-yl 3',4'-bisbenzyloxybenzoate and 0.3 g of 10 % Pd-C in dry tetrahydrofuran was stirred for 6 hours at room temperature in an $H_2$ atmosphere. Separated precipitates were filtered off and the filtrate was concentrated under reduced pressure to give 0.270 g of 5-carbamoyl-1H-imidazole-4-yl 3',4'-dihydroxybenzoate. m.p.: 156°C.

Crude material was recrystallized from dimethylsulfoxide and water. m.p.: 159 - 161°C.

$\nu_{max}^{nujol}$ ($cm^{-1}$): 3480, 3225, 1735, 1675, 1600

Elemental analysis:

| | C(%) | H(%) | N(%) |
|---|---|---|---|
| Calculated for $C_{11}H_9N_3O_5 \cdot 1.2H_2O$ | 46.39 | 4.03 | 14.75 |
| Found | 46.46 | 3.81 | 14.45 |

## Example 6

A mixture of 0.212 g of 3,5-dinitrobenzoic acid, 0.127 g of 4-carbamoylimidazolium-5-olate and 0.206 g of dicyclohexylcarbodiimide in 4 ml of dry pyridine was stirred for 21 hours at room temperature. Separated precipitates were filtered off and were washed with ethyl acetate to give 0.355 g of 5-carbamoyl-1H-imidazole-4-yl 3',5'-dinitrobenzoate which was recrystallized from dimethylsulfoxide and water. m.p.: 220°C. (decomp.)

Elemental analysis:

| | C(%) | H(%) | N(%) |
|---|---|---|---|
| Calculated for $C_{11}H_7N_5O_7$ | 41.13 | 2.20 | 21.81 |
| Found | 41.5 | 2.4 | 20.9 |

## Example 7

Following a procedure similar to that of Example 6 but using 0.83 g of 3-methoxy-4-methylbenzoic acid, 1.03 g of dicyclohexylcarbodiimide and 0.635 g of 4-carbamoylimidazolium-5-olate there was obtained 1.45 g of 5-carbamoyl-1H-imidazole-4-yl 3'-methoxy-4'-methylbenzoate.

Crude material was recrystallized from N,N-dimethylformamide and water. m.p.: 210°C. (decomp.)

$\nu_{max}^{nujol}$ ($cm^{-1}$): 3450, 1740, 1655, 1600

## Example 8

A mixture of 76.26 g of 4-carbamoylimidazolium-5-olate, 174.31 g of 1,1,1,3,3,3-hexamethyldisilazane, 1.59 g of ammonium sulfate and 500 g of dry xylene was refluxed for 4 hours. The reaction mixture was concentrated under reduced pressure and a tris-trimethylsilylated derivative of 4-carbamoylimidazolium-5-olate was obtained.

m.p.: 83 - 86.5°C.

To a stirred solution of 1.718 g of tris-trimethylsilylated derivative of 4-carbamoyl-imidazolium-5-olate in 15 ml of dry tetrahydrofuran was added dropwise a solution of 0.833 g of cinnamoyl chloride in 5 ml of dry tetrahydrofuran at -50°C. under an $N_2$ atmosphere. After stirring the mixture for ½ hr. at -50°C., 0.32 g of dry methanol was added, and after further stirring for 15 minutes at -50°C., 0.51 g of triethylamine was added.

The reaction mixture was heated up to room temperature and then separated crystals were filtered off, washed with tetrahydrofuran and chloroform and dried to give 0.796 g of 5-carbamoyl-1H-imidazole-4-yl cinnamate. m.p.: 170 - 175°C. The filtrate of a tetrahydrofuran solution was concentrated and diethylether was added to the residue and separated crystals were filtered off, washed with diethyl ether and dried to give 0.427 g of product.

Crude material was recrystallized from dimethylsulfoxide and water. m.p.: 197°C. (charred)

$\nu_{max}^{nujol}$ ($cm^{-1}$): 3460, 3160, 3110, 1720, 1670, 1605

Elemental analysis:

| | C(%) | H(%) | N(%) |
|---|---|---|---|
| Calculated for $C_{13}H_{11}N_3O_3 \cdot 0.1H_2O$ | 60.27 | 4.36 | 16.22 |
| Found | 60.1. | 4.4 | 16.3 |

Example 9

Following a procedure similar to that of Example 8 but using 0.9 g of p-methylcinnamoyl chloride there was obtained 1.270 g of 5-carbamoyl-1H-imidazole-4-yl p-methyl-

0075629

cinnamate. m.p.: 171°C. (decomp.)

Crude material was recrystallized from dimethylsulfoxide and water. m.p.: 194°C. (charred)

$\nu_{max}^{nujol}$ ($cm^{-1}$): 3440, 3150, 1715, 1665, 1600

Elemental analysis:

| | C(%) | H(%) | N(%) |
|---|---|---|---|
| Calculated for $C_{14}H_{13}N_3O_3 \cdot 0.2H_2O$ | 61.17 | 4.91 | 15.29 |
| Found | 61.19 | 4.69 | 15.49 |

Example 10

Following a procedure similar to that of Example 8 but using 1.01 g of p-chlorocinnamoyl chloride there was obtained 1.408 g of 5-carbamoyl-1H-imidazole-4-yl p-chlorocinnamate. m.p. 172°C. (dec.)

Crude material was recrystallized from dimethylsulfoxide and water. m.p.: 202°C. (dec.)

$\nu_{max}^{nujol}$ ($cm^{-1}$): 3440, 3380, 3150, 1725, 1660, 1615

Elemental anlaysis:

| | C(%) | H(%) | N(%) |
|---|---|---|---|
| Calculated for $C_{13}H_{10}N_3O_3Cl \cdot 0.1H_2O$ | 53.20 | 3.5 | 14.32 |
| Found | 53.1 | 3.8 | 14.1 |

Example 11

Following a procedure similar to that of Example 8 but using 1.053 g of 3,4-methylenedioxycinnamoyl chloride there was obtained 1.172 g of 5-carbamoyl-1H-imidazole-4-yl 3',4'-methylenedioxycinnamate. m.p.: 165 - 175°C.

Crude material was recrystallized from dimethylsulfoxide and water. m.p.: 189°C. (char.)

$\nu_{max}^{nujol}$ ($cm^{-1}$): 3460, 3400, 3125, 1735, 1670, 1630, 1605

Elemental analysis:

| | C(%) | H(%) | N(%) |
|---|---|---|---|
| Calculated for $C_{14}H_{11}N_3O_5 \cdot 0.4H_2O$ | 54.51 | 3.86 | 13.62 |
| Found | 54.49 | 3.71 | 13.48 |

## Example 12

Following a procedure similar to that of Example 8 but using 0.98 g of p-methoxycinnamoyl chloride, there was obtained 1.15 g of 5-carbamoyl-1H-imidazole-4-yl p-methoxycinnamate. m.p.: 165 - 167°C. (dec.)

Crude material was recrystallized from dimethylsulfoxide and water. m.p.: 185°C. (char.)

$\nu_{max}^{nujol}$ ($cm^{-1}$): 3470, 3175, 1725, 1670, 1605

Elemental analysis:

| | C(%) | H(%) | N(%) |
|---|---|---|---|
| Calculated for $C_{14}H_{13}N_3O_4 \cdot 0.6H_2O$ | 56.41 | 4.80 | 14.10 |
| Found | 56.75 | 4.63 | 13.85 |

## Example 13

Following a procedure similar to that of Example 8 but using 0.97 g of p-formylcinnamoyl chloride, there was obtained 1.086 g of 5-carbamoyl-1H-imidazole-4-yl p-formylcinnamate.

Crude material was recrystallized from dimethylsulfoxide and water. m.p.: 233°C. (char.)

$\nu_{max}^{nujol}$ ($cm^{-1}$): 3100, 1730, 1680, 1650, 1620, 1590

Elemental analysis:

| | C(%) | H(%) | N(%) |
|---|---|---|---|
| Calculated for $C_{14}H_{11}N_3O_4 \cdot 0.5H_2O$ | 57.14 | 4.11 | 14.28 |
| Found | 57.0 | 3.9 | 14.0 |

Example 14

Following a procedure similar to that of Example 8 but using 1.173 g of m-trifluoromethylcinnamoyl chloride, there was obtained 1.236 g of 5-carbamoyl-1H-imidazole-4-yl m-trifluoromethylcinnamate. m.p.: 167 - 170°C. (dec.)

Crude material was recrystallized from dimethylsulfoxide and water. m.p.: 188°C. (char.)

$\nu_{max}^{nujol}$ ($cm^{-1}$): 3460, 3175, 1730, 1680, 1610

Elemental analysis:

| | C(%) | H(%) | N(%) |
|---|---|---|---|
| Calculated for $C_{14}H_{10}N_3O_3F_3 \cdot 0.1H_2O$ | 51.42 | 3.14 | 12.85 |
| Found | 51.19 | 2.98 | 13.09 |

Example 15

Following a procedure similar to that of Example 8 but using 1.058 g of m-nitrocinnamoyl chloride, there was obtained 1.383 g of 5-carbamoyl-1H-imidazole-4-yl m-nitrocinnamate.

Crude material was recrystallized from dimethylsulfoxide and water. m.p.: 197°C. (char.)

$\nu_{max}^{nujol}$ ($cm^{-1}$): 3450, 3120, 1740, 1650, 1600

Elemental analysis:

| | C(%) | H(%) | N(%) |
|---|---|---|---|
| Calculated for $C_{13}H_{10}N_4O_5 \cdot 0.3H_2O$ | 50.75 | 3.47 | 18.21 |
| Found | 50.76 | 3.30 | 18.20 |

Example 16

Following a procedure similar to that of Example 1 but using 1.46 g of α-phenylcinnamoyl chloride, there was obtained 1.186 g of 5-carbamoyl-1H-imidazole-4-yl α-phenylcinnamate. m.p.: 208°C.

Crude material was recrystallized from dimethylsulfoxide and water. m.p.: 212°C.

$\nu_{max}^{nujol}$ (ck$^{-1}$): 3450, 3125, 1735, 1655, 1605

Elemental analysis:

| | C(%) | H(%) | N(%) |
|---|---|---|---|
| Calculated for $C_{19}H_{15}N_3O_3 \cdot 0.3H_2O$ | 67.37 | 4.64 | 12.40 |
| Found | 67.48 | 4.51 | 12.41 |

Example 17

Following a procedure similar to that of Example 1 but using 2.544 g of 4-carbamoylimidazolium-5-olate and 5.922 g of p-benzyloxybenzoyl chloride, there was obtained 5-carbamoyl-1H-imidazole-4-yl p-benzyloxybenzoate.

Crude material was recrystallized from dimethylsulfoxide and water. m.p.: 215 - 218°C. (dec.)

$\nu_{max}^{nujol}$ (cm$^{-1}$): 1730, 1680

Elemental analysis:

| | C(%) | H(%) | N(%) |
|---|---|---|---|
| Calculated for $C_{18}H_{15}O_4N_3$ | 64.09 | 4.48 | 12.46 |
| Found | 63.5 | 4.5 | 12.3 |

Example 18

Following a procedure similar to that of Example 1 but using m-trifluoromethylbenzoyl chloride, there was obtained 5-carbamoyl-1H-imidazole-4-yl m-trifluoromethylbenzoate. m.p. 208 - 211°C. (dec.)

$\nu_{max}^{nujol}$ (cm$^{-1}$): 1730, 1670

Elemental analysis:

| | C(%) | H(%) | N(%) |
|---|---|---|---|
| Calculated for $C_{12}H_8O_3N_3F_3$ | 48.17 | 2.7 | 14.04 |
| Found | 48.0 | 2.9 | 14.0 |

Example 19

Following a procedure similar to that of Example 1 but using p-N-benzyloxycarbonylaminobenzoyl chloride, there was obtained 5-carbamoyl-1H-imidazole-4-yl p-N-benzyloxycarbonylaminobenzoate. m.p.: 187 - 190°C. (dec.)

$\nu_{max}^{nujol}$ ($cm^{-1}$): 1730, 1680, 1660

Elemental Analysis:

| | C(%) | H(%) | N(%) |
|---|---|---|---|
| Calculated for $C_{19}H_{16}O_5N_4$ | 59.99 | 4.24 | 14.73 |
| Found | 59.6 | 4.3 | 14.5 |

Example 20

Following a procedure similar to that of Example 1 but using 1.41 g of 4-biphenylcarbonyl chloride, there was obtained 1.33 g of 5-carbamoyl-1H-imidazole-4-yl p-henylbenzoate. m.p.: 225°C. (char.)

Crude material was recrystallized from N,N-dimethylformamide and water. m.p.: 226.5 - 227°C. (char.)

$\nu_{max}^{nujol}$ ($cm^{-1}$): 3460, 3160, 1735, 1675, 1605

Elemental analysis:

| | C(%) | H(%) | N(%) |
|---|---|---|---|
| Calculated for $C_{17}H_{13}N_3O_3$ | 66.44 | 4.26 | 13.67 |
| Found | 66.6 | 4.3 | 13.5 |

Example 21

Following a procedure similar to that of Example 8 but using 1.1 g of o-acetylsalicyloyl chloride, there was obtained 1.29 g of 5-carbamoyl-1H-imidazole-4-yl o-acetoxybenzoate. m.p.: 143-145°C.

$\nu_{max}^{nujol}$ ($cm^{-1}$): 3430, 3190, 1755, 1670, 1605

Example 22

To a stirred solution of 6.3 ml of N,N-dimethylformamide in 14 ml of acetonitrile was added slowly 0.45 ml of oxalyl chloride at -20°C. and the reaction mixture was heated up to room temperature. To the reaction mixture was added 1.33 g of o-benzyloxycarbonylbenzoic acid at -25°C. and the reaction mixture was heated up to room temperature. To the reaction mixture were added 0.636 g of 4-carbamoylimidazolium-5-olate and 2.5 ml of dry pyridine over ice bath in an $N_2$ atmosphere and stirring was continued for 30 minutes at room temperature.

To the residue was added 1.9 ml of triethylamine over an ice bath and separated precipitates were filtered off and the filtrate was concentrated under reduced pressure to give 0.78 g of 5-carbamoyl-1H-imidazole-4-yl o-benzyloxycarbonylbenzoate. m.p.: 122 - 124°C.

$\nu_{max}^{nujol}$ ($cm^{-1}$): 3460, 3150, 1770, 1725, 1670, 1610

Example 23

Following a procedure similar to that of Example 1 but using 1.19 g of p-methoxycarbonylbenzoyl chloride, there was obtained 1.4 g of 5-carbamoyl-1H-imidazole-4-yl p-methoxycarbonylbenzoate.

Crude material was recrystallized from dimethylsulfoxide and water. m.p.: 215°C. (dec.)

$\nu_{max}^{nujol}$ ($cm^{-1}$): 3475, 3440, 3180, 3120, 1750, 1735, 1680, 1605

Elemental analysis:

| | C(%) | H(%) | N(%) |
|---|---|---|---|
| Calculated for $C_{13}H_{11}N_3O_5$ | 53.98 | 3.83 | 14.53 |
| Found | 53.44 | 3.80 | 14.46 |

According to the present invention, there are obtained, for example, the following compounds:

5-carbamoyl-1H-imidazole-4-yl p-aminocinnamate,
5-carbamoyl-1H-imidazole-4-yl p-hydroxycinnamate,
5-carbamoyl-1H-imidazole-4-yl o-carboxycinnamate,
5-carbamoyl-1H-imidazole-4-yl α-cyano-4'-hydroxycinnamate,
5-carbamoyl-1H-imidazole-4-yl 3',4'-dichlorocinnamate,
5-carbamoyl-1H-imidazole-4-yl 3',4'-dimethoxycinnamate,
5-carbamoyl-1H-imidazole-4-yl 3',4'-dimethylcinnamate,
5-carbamoyl-1H-imidazole-4-yl 4'-hydroxy-3'-methoxycinnamate,
5-carbamoyl-1H-imidazole-4-yl 3',4',5'-trimethoxycinnamate,
5-carbamoyl-1H-imidazole-4-yl 3',4'-dihydroxycinnamate,
5-carbamoyl-1H-imidazole-4-yl α-fluorocinnamate,
5-carbamoyl-1H-imidazole-4-yl 3',5'-dimethoxy-4'-hydroxycinnamate,
5-carbamoyl-1H-imidazole-4-yl α-methylcinnamate,
5-carbamoyl-1H-imidazole-4-yl p-octylcinnamate,
5-carbamoyl-1H-imidazole-4-yl p-dodecylcinnamate,
5-carbamoyl-1H-imidazole-4-yl p-docosylcinnamate,
5-carbamoyl-1H-imidazole-4-yl p-octyloxycinnamate,
5-carbamoyl-1H-imidazole-4-yl p-octadecyloxycinnamate,
5-carbamoyl-1H-imidazole-4-yl pentafluorobenzoate,
5-carbamoyl-1H-imidazole-4-yl 2',4'-dicarboxybenzoate,
5-carbamoyl-1H-imidazole-4-yl 3'-amino-4'-methylbenzoate,
5-carbamoyl-1H-imidazole-4-yl 3'-acetyloxy-4'-methylbenzoate,
5-carbamoyl-1H-imidazole-4-yl 2'-benzyloxy-5'-methylthiobenzoate,
5-carbamoyl-1H-imidazole-4-yl 4'-methylsulfonyl-3'-nitrobenzoate,

5-carbamoyl-1H-imidazole-4-yl 4'-chloro-3'-sulfamoylbenzoate,
5-carbamoyl-1H-imidazole-4-yl 2'-hydroxy-5'-methylthiobenzoate,
5-carbamoyl-1H-imidazole-4-yl 5'-formyl-2'-hydroxybenzoate,
5-carbamoyl-1H-imidazole-4-yl 2'-hydroxy-5'-sulfobenzoate,
5-carbamoyl-1H-imidazole-4-yl 4'-diethylamino-2'-hydroxybenzoate,
5-carbamoyl-1H-imidazole-4-yl 4'-methyl-3'-octyloxybenzoate,
5-carbamoyl-1H-imidazole-4-yl 4'-methyl-3'-octodecyloxybenzoate,
5-carbamoyl-1H-imidazole-4-yl 3'-methoxy-4'-lauroyloxybenzoate,
5-carbamoyl-1H-imidazole-4-yl 3'-methoxy-4'-stearoyloxybenzoate,
5-carbamoyl-1H-imidazole-4-yl 4'-hexadecyl-2'-methylbenzoate,
5-carbamoyl-1H-imidazole-4-yl 2'-dodecyl-4'-methoxybenzoate,
5-carbamoyl-1H-imidazole-4-yl p-aminobenzoate,
5-carbamoyl-1H-imidazole-4-yl p-hydroxybenzoate,
5-carbamoyl-1H-imidazole-4-yl o-benzoylbenzoate,
5-carbamoyl-1H-imidazole-4-yl o-carboxybenzoate,
5-carbamoyl-1H-imidazole-4-yl o-phenoxybenzoate,
5-carbamoyl-1H-imidazole-4-yl p-formylbenzoate,
5-carbamoyl-1H-imidazole-4-yl p-acetylbenzoate,
5-carbamoyl-1H-imidazole-4-yl p-carbamoylbenzoate,
5-carbamoyl-1H-imidazole-4-yl p-octanoylbenzoate,
5-carbamoyl-1H-imidazole-4-yl p-palmitoylbenzoate,
5-carbamoyl-1H-imidazole-4-yl p-octyloxycarbonylbenzoate,
5-carbamoyl-1H-imidazole-4-yl p-octadecyloxycarbonylbenzoate,
5-carbamoyl-1H-imidazole-4-yl p-octanoyloxybenzoate,
5-carbamoyl-1H-imidazole-4-yl p-palmitoyloxybenzoate.

CLAIMS:

1. A compound of the formula:

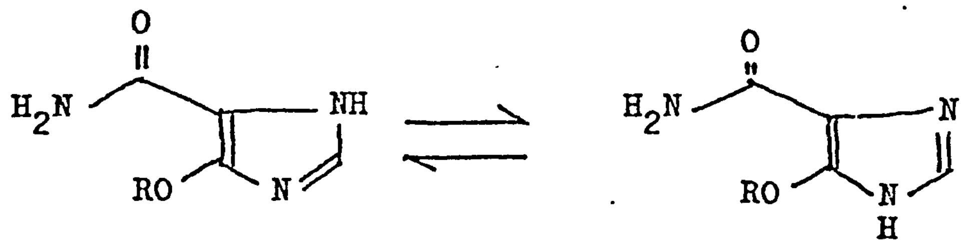

wherein R is a monosubstituted benzoyl group in which the substitutent is (a) a phenyl, alkanoyl, formyl, halogenoalkyl, aralkyloxy, phenoxy, alkoxycarbonyl, aralkyloxycarbonyl, alkanoyloxy, benzoyl, carboxyl, hydroxy, aralkyloxycarbonylamino, lower alkoxycarbonylamino, carboxyamino or carbamoyl group or a group (IV)

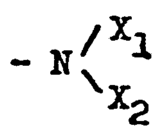

(wherein $X_1$ and $X_2$ are each independently hydrogen or lower alkyl); (b) a cinnamoyl group which may be substituted at the α-position and/or the phenyl ring with one or more substitutents selected independently from alkyl, alkoxy, aryl, nitro, methylenedioxy, formyl, halogenoalkyl, hydroxy, carboxyl, amino and cyano groups and halogens or (c) a benzoyl group substituted with from two to five substituents selected independently from alkyl, alkoxy, aralkyloxy, nitro, hydroxy, alkanoyloxy, formyl, carboxyl, alkylthio, alkylsulfonyl, sulfo and sulfamoyl groups, halogens, and groups (IV) above; and non-toxic salts thereof.

2. A compound according to claim 1 wherein R is a monosubstituted benzoyl group.

3. A compound according to claim 1 wherein R is a substituted or unsubstituted cinnamoyl group.

4. A compound according to claim 1 wherein R is a benzoyl group substituted with from two to five of the same or different substituents.

5. A compound according to claim 2 wherein R is selected from p-benzyloxybenzoyl, m-trifluoromethylbenzoyl, p-N-benzyloxycarbonylaminobenzoyl, 4-biphenylylcarbonyl, o-acetylsalicyloyl, o-benzyloxycarbonylbenzoyl and p-methoxycarbonylbenzoyl.

6. A compound according to claim 3 wherein R is selected from cinnamoyl, p-methylcinnamoyl, p-chlorocinnamoyl, 3,4-methylenedioxycinnamoyl, p-methoxycinnamoyl, p-formylcinnamoyl, m-trifluoromethylcinnamoyl, m-nitrocinnamoyl and α-phenylcinnamoyl.

7. A compound according to claim 4 wherein R is selected from 2,6-dimethylbenzoyl, 3,4-dimethoxybenzoyl, 2,4,6-trimethoxybenzoyl, 3,4-bisbenzyloxybenzoyl, 3,4-dihydroxybenzoyl, 3,5-dinitrobenzoyl and 3-methoxy-4-methylbenzoyl.

8. A process for producing a compound according to claim 1 which comprises reacting 4-carbamoylimidazolium-5-olate :

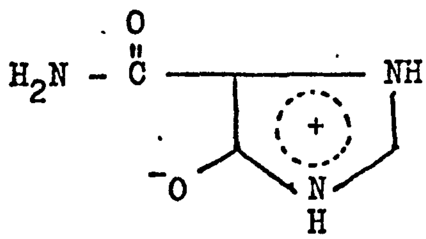

or reactive derivative thereof with carboxylic acid of the formula :

R - OH

or reactive derivative thereof where R is as defined in claim 1.

9. An antitumor composition which comprises an antitumor effective amount of a compound or compounds according to any of claims 1 to 7 as active ingredient and a pharmaceutically acceptable carrier or diluent.

10. An immunosuppressant composition which comprises an immunosuppressive amount of compound or compounds according to any of claims 1 to 7 as active ingredient and a pharmaceutically acceptable carrier or diluent.

European Patent Office

# EUROPEAN SEARCH REPORT

0075629
Application number

EP 81 30 4514

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
| --- | --- | --- | --- |
| A | GB-A-1 567 662 (SUMITOMO)<br>--- | | C 07 D 233/90<br>A 61 K 31/415 |
| A | BE-A- 876 625 (SUMITOMO)<br>----- | | |
| | | | TECHNICAL FIELDS SEARCHED (Int. Cl. 3) |
| | | | C 07 D 233/00<br>A 61 K 31/00 |

The present search report has been drawn up for all claims

| Place of search | Date of completion of the search | Examiner |
| --- | --- | --- |
| THE HAGUE | 24-05-1982 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document